(19) Europäisches Patentamt
European Patent Office
Office européen des brevets

(11) Numéro de publication: 0 124 420 B1

(12) **FASCICULE DE BREVET EUROPEEN**

(45) Date de publication du fascicule du brevet: **19.11.87**

(51) Int. Cl.⁴: **C 07 K 7/06**, A 61 K 37/02

(21) Numéro de dépôt: **84400787.2**

(22) Date de dépôt: **19.04.84**

(54) **Nouveaux dérivés peptidiques inhibiteurs de la sécrétion gastrique, procédé pour leur préparation et médicaments les contenant.**

(30) Priorité: **20.04.83 FR 8306492**

(43) Date de publication de la demande: **07.11.84 Bulletin 84/45**

(45) Mention de la délivrance du brevet: **19.11.87 Bulletin 87/47**

(84) Etats contractants désignés: **AT BE CH DE FR GB IT LI LU NL SE**

(56) Documents cités:
**FR - M - 5 336**

**UNLISTED DRUGS**, *vol. 26, no. 6*, **1974**, *page* **86**, *point b:* **"CTO"**

(73) Titulaire: **SANOFI, société anonyme, 40, Avenue George V, F-75008 Paris (FR)**
Titulaire: **Etablissement Public dit: CENTRE NATIONAL DE LA RECHERCHE SCIENTIFIQUE (CNRS), 15, Quai Anatole France, F-75007 Paris (FR)**

(72) Inventeur: **Martinez, Jean, Rue des Cévennes, F-34720 Caux (FR)**
Inventeur: **Bali, Jean-Pierre, 278 rue du Bosquet, F-34980 Saint-Gely du Fesc (FR)**
Inventeur: **Castro, Bertrand, L'Estaple Avenue des Costières, F-34130 Saint-Aunes (FR)**
Inventeur: **Nisato, Dino, Viale Colgi, I-8000 Pavia (IT)**
Inventeur: **Demarne, Henri, Le Florence Avenue Major Flandre, F-34000 Montpellier (FR)**

(74) Mandataire: **Gillard, Marie-Louise et al, Cabinet Beau de Loménie 55, Rue d'Amsterdam, F-75008 Paris (FR)**

EP 0 124 420 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La caeruleine, la cholécystokinine et la gastrine sont des polypeptides naturels doués d'activités pharmacologiques diverses.

Ainsi la gastrine stimule la sécrétion gastrique, la cholécystokinine stimule la libération d'enzymes pancréatiques alors que la caéruleine augmente les sécrétions gastriques, pancréatiques et biliaires.

Sur le plan structural ces 3 composés possèdent la particularité de présenter la même séquence C-terminale qui, en utilisant pour désigner les α-aminoacides les abréviations à 3 lettres recommandées par la Commission de Nomenclature de l'IUPAC-IUB, peut être représentée par:

$$-Trp-Met-Asp-Phe-NH_2$$

Selon la présente invention, il a été trouvé que des composés peptidiques dérivés des séquences C-terminale de la caéruleine, de la cholécystokinine et da la gastrine par suppression du groupe Phe $NH_2$ terminal possèdent de façon surprenante la propriété d'inhiber la sécrétion gastrique.

Ces composés répondent à la formule générale où tous les aminoacides sont de configuration L:

$$R-A-Gly-Trp-B-Asp-NH_2 \qquad (I)$$

dans laquelle:

– Asp $NH_2$ représente l'amide en α de l'acide aspartique de formule

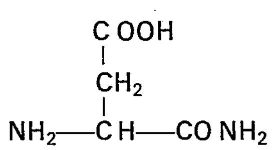

– R représente l'hydrogène, un groupe protecteur de la fonction amine terminale tel que t.butyloxy carbonyl (BOC), benzyloxy carbonyl (Z), alcanoyle inférieur, etc.

– A désigne:

– soit la tyrosine, la tyrosine-O-sulfate, la thréonine ou la méthionine;

– soit un dipeptide choisi parmi: – Ala – TYR –, – TYR – Thr–, – TYR – Met –, en désignant par TYR l'ensemble des 2 amino-acides constitués par la tyrosine ou la tyrosine-O-sulfate;

– soit un tripeptide choisi parmi: – Glu – Ala – TYR –, Asp – TYR – Thr –, – Asp – TYR – Met –;

– soit un tétrapeptide choisi parmi: – Glu – Glu – Ala – TYR –, – Gln – Asp – TYR – Thr –, – Arg – Asp – TYR – Met –;

– soit un pentapeptide choisi parmi: – Glu – Glu – Glu – Ala – TYR –, – Pyr – Gln – Asp – TYR – Thr –,

– Asp – Arg – Asp – TYR – Met –.

– B désigne la méthionine, la leucine ou la norleucine.

Les composés selon l'invention peuvent être préparés par les techniques habituelles de synthèse peptidique soit en phase solide selon Merrifield, soit en phase liquide.

A partir de l'acide aspartique α-amide, on introduit successivement les différents aminoacides présents dans la séquence. Les réactions de couplage sont effectuées avec un ester activé de l'acide aminé à introduire au sein du diméthylformamide et en présence de diisopropyléthylamine et d'hydroxy-1-benzotriazole.

Tous les aminoacides sont incorporés sous la forme du dérivé protégé sur l'amine en α, le groupe protecteur étant choisi parmi les groupes benzyloxycarbonyle et t-butyloxycarbonyle. Lorsque l'aminoacide utilisé présente dans sa chaîne latérale des fonctions susceptibles de réagir, celles-ci doivent être bloquées au préalable. Ainsi les fonctions acides en ε de l'acide aspartique ou de l'acide glutamique doivent être bloquées sous forme d'ester en particulier l'ester benzylique ou l'ester tertiobutylique et le groupe guanidino de l'arginine peut être protégé par un groupe nitro.

Après chaque réaction de couplage, la déprotection de l'amine en α est effectuée soit par hydrogénolyse s'il s'agit d'un groupe benzyloxy carbonyle, soit par hydrolyse acide s'il s'agit d'un groupe butyloxycarbonyle.

Finalement, les peptides protégés dans leurs fonctions des chaînes latérales sont partiellement ou entièrement déprotégés pour conduire aux composés de formule (I).

Les composés de formule (I), où TYR représente la tyrosine-O-sulfate, sont obtenus à partir des composés où TYR représente la tyrosine par action de l'anhydride sulfurique en présence de pyridine.

Les exemples suivants non limitatifs permettront de mieux comprendre l'invention.

Dans ces exemples, on utilisera les abréviations suivantes:

Acides aminés et groupes protecteurs:

| | |
|---|---|
| Ala | : Alanine |
| Asp $NH_2$ | : Acide aspartique α-amide |
| Met | : Méthionine |
| Trp | : Tryptophane |
| Glu | : Acide glutamique |
| Gly | : Glycine |
| Tyr | : Tyrosine |
| Tyr ($SO_3H$) | : Tyrosine-O-sulfate |

A l'exception de la glycine, tous ces acides aminés sont de configuration L.

| | |
|---|---|
| Boc | : Tertiobutyloxycarbonyl |
| Z | : Benzyloxycarbonyl |
| Bzl | : Benzyle |
| O bu$^t$ | : O-tertiobutyl |
| CAM | : carboxamidométhyl |
| FMOC | : fluorényl-méthyl-oxycarbonyl |

ONSu 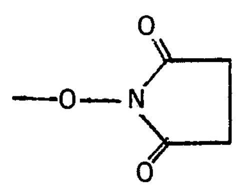

ONp 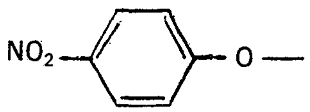

On utilisera également les abréviations suivantes:

| | |
|---|---|
| DIEA | : Diisopropyléthylamine |
| HOBt | : Hydroxy-1-benzotriazole |
| DOP | : hexafluorphosphate de benzotriazolyloxy tris diméthylaminophosphonium |

Exemple 1

H – Met – Gly – Trp – Met – Asp $NH_2$

(I) R = H; A = Met; B = Met

a) – Z – Asp (β O $Bu^t$) – $NH_2$

On dissout 5 g d'acide N-benzyloxycarbonyl aspartique α ester de N-hydroxy succinimide β ester de tertiobutyle dans un mélange de 100 ml d'acétate d'éthyle et 100 ml de dichlorométhane. On fait passer un courant d'ammoniac gazeux au-dessus de la solution bien agitée durant 1 heure.

Par évaporation, on obtient le produit attendu. F: 88–92 °C; Rendement: 95%.

b) – Z – Met – Asp (β O $Bu^t$) – $NH_2$

La solution de 3,5 g du composé obtenu en a) dans l'éthanol absolu est additionnée de 2 ml d'acide chlorhydrique et 0,35 g de palladium sur charbon à 10%. On hydrogène pendant 4 heures puis on filtre le catalyseur et évapore le solvant. Le résidu est lavé plusieurs fois avec de l'éther, puis il est ajouté à une solution de 2,7 g de Z – Met – ONp, 2,3 ml de DIEA et 0,9 g d'HOBt dans le diméthylformamide.

Après 24 heures, on évapore le solvant sous vide et reprend le résidu dans le dichlorométhane. On lave la solution avec de l'eau plusieurs fois avec une solution d'acide chlorhydrique à 5% et avec une solution saturée de bicarbonate de sodium. On évapore à siccités sous vide et obtient le produit attendu.

F: 155–157 °C; Rendement: 80%.

$[\alpha]_D^{25} = -3{,}9$ (c=1, diméthylformamide).

c) – Z – Trp – Met – Asp (β O $Bu^t$) – $NH_2$

2,2 g du composé obtenu ci-dessus sont dissous dans un mélange de 150 ml de diméthylformamide, 30 ml d'eau et 15 ml de DIEA. On ajoute 0,4 g de palladium sur sulfate de baryum à 10% et hydrogène pendant une nuit.

On filtre le catalyseur, et concentre les solvants sous vide. On dissout le résidu dans le diméthylformamide (100 ml) et ajoute 1,8 g de Z – Trp – ONp, 1,6 g de DIEA et 0,75 g d'HOBt.

On laisse une nuit en contact puis on concentre à environ 50 ml et filtre sur 30 g d'alumine neutre. On poursuit l'élution avec 200 ml de diméthylformamide, évapore à siccité et triture le résidu avec de l'acétate d'éthyle.

On obtient une poudre blanche.

$[\alpha]_D^{25} = -30$ (c=1, diméthylformamide); Rendement: 82%.

d) – Z – Gly – Trp – Met – Asp (β O $Bu^t$) – $NH_2$

A partir du composé obtenu ci-dessus, on opère comme indiqué dans le paragraphe c) en remplaçant Z – Trp – ONp par une quantité équivalente de Z – Gly – ONp.

On obtient de la même façon une poudre blanche. F: 187–189 °C; Rendement: 75%.

$[\alpha]_D^{25} = -18$ (c=1, diméthylformamide).

e) – Boc – Met – Gly – Trp – Met – Asp (β O $Bu^t$) – $NH_2$

A partir du composé obtenu au paragraphe d), on opère comme en c) en remplaçant Z – Trp – ONp par une quantité équivalente de Boc – Met – ONp. On isole de la même façon le produit attendu.

F: 194 °C (dec); Rendement: 75%.

f) – H – Met – Gly – Trp – Met – Asp – $NH_2$

Le peptide protégé obtenu ci-dessus est dissous dans l'acide trifluoracétique à raison de 5 ml par g de produit et on ajoute 0,5 ml d'anisole et 0,5 ml de thioanisole. On laisse en contact pendant 2 heures puis on ajoute 200 ml d'éther. On essore le précipité qui est lavé plusieurs fois avec de l'éther.

On obtient le trifluoracétate sous forme de solide incolore.

Rendement: 85%.

Exemple 2

Z – Tyr – Met – Gly – Trp – Met – Asp – $NH_2$

(I) R = Z; A = – Tyr – Met; B = Met

On dissout 1,8 g du trifluoracétate obtenu à l'exemple 1 f) dans le diméthylformamide, puis on ajoute 1,12 g de Z – Tyr – ONp et 0,71 g de DIEA et laisse 12 heures en contact.

On ajoute de l'acétate d'éthyle et essore le précipité qu'on lave plusieurs fois avec de l'acétate d'éthyle puis avec de l'éther.

On chromatographie sur gel de silice et, en éluant avec le mélange acétate d'éthyle-pyridine-acide acétique-eau (60–20–6–10 vol/vol), on obtient le produit attendu avec un rendement de 80%.

Exemple 3

Z – Tyr ($SO_3H$) – Met – Gly – Trp – Met – Asp – $NH_2$

(I) R = Z; A = – Tyr ($SO_3H$) – Met; B = Met

On dissout 0,1 g du peptide obtenu à l'exemple 2 dans 3 ml de diméthylformamide et 3 ml de pyridine. On ajoute 0,75 g du complexe anhydride sulfurique/pyridine et agite durant une nuit.

On évapore les solvants sous vide et amène le pH à 6,5–7 par addition de solution saturée de bicarbonate de sodium. On laisse 1 heure en contact puis on isole le précipité qui est purifié par chromatographie sur gel de silice de façon analogue à la purification du peptide de l'exemple 2.

On obtient le produit attendu avec un rendement de 55%. F: 260 °C (déc).

Spectre IR: une bande à 1060 $cm^{-1}$ due au sulfate.

Rf = 0,3 (acétate d'éthyle 60 – pyridine 20 – acide acétique 6 – eau 10, vol/vol).

Exemple 4

Boc – Tyr – Gly – Trp – Met – Asp – $NH_2$

(I) R = Boc; A = Tyr; B = Met

a) – Boc – Met – Asp (β O Bzl) – $NH_2$

On dissout 1,91 g de trifluoracétate de Asp (β O Bzl) – $NH_2$ dans 10 ml de diméthylformamide et on ajoute 2,07 g de Boc – Met – ONp et 0,76 g d'HOBt.

On refroidit la solution à 5 °C et ajoute 2,06 ml de DIEA. Après 8 heures, on concentre sous vide et reprend le résidu huileux dans l'acétate d'éthyle (200 ml). On lave la solution organique avec une solution saturée de bicarbonate de sodium (2 fois 50 ml), avec de l'eau (1 fois 50 ml), avec une solution saturée de chlorure de sodium (1 fois 50 ml), avec une solution d'acide citrique à 20% (1 fois 50 ml) puis à nouveau avec de l'eau (1 fois 50 ml) et avec une solution saturée de chlorure desodium et concentre le solvant sous vide à température inférieure à 50 °C.

Le résidu, lavé plusieurs fois avec le mélange acétate d'éthyle-éther (1–9, vol/vol), puis avec de l'éther, cristallise.

F: 109–111 °C; Rendement: 85%.

Chromatographie en couche mince:

Rf = 0,4 (chloroforme 7 – hexane 3 vol/vol)

Rf = 0,3 (acétate d'éthyle 8 – hexane 2 vol/vol).

b) – Boc – Trp – Met – Asp (β O Bzl) – $NH_2$

2 g du composé obtenu ci-dessus sont dissous dans 5 ml d'acide trifluoracétique. On laisse 1/2 heure puis on ajoute 100 ml d'éther en agitant. On essore le précipité qui s'est formé, on le rince plusieurs fois avec de l'éther puis on le sèche sur potasse.

On dissout le produit dans 10 ml de diméthylformamide et on ajoute 1,7 g de Boc – Trp – ONp et 0,56 g d'HOBt. Après avoir refroidi à 5 °C, on ajoute 1,47 ml de DIEA.

On laisse 8 heures et traite comme indiqué en a). On obtient un solide, F: 178–180 °C; Rendement: 86%.

Chromatographie en couche mince:

Rf = 0,35 (chloroforme 7 – hexane 3 vol/vol)

Rf = 0,2 (acétate d'éthyle 8 – hexane 2, vol/vol).

c) – Boc – Gly – Trp – Met – Asp (β O Bzl) – $NH_2$

On élimine le groupe Boc terminal du peptide obtenu au paragraphe b) par action de l'acide trifluoracétique. 1 g du trifluoracétate obtenu est mis en solution dans le diméthylformamide et on ajoute 0,48 g de Boc – Gly – ONp, 0,22 g de HOBt et 0,6 g de DIEA. Après 8 heures de réaction on concentre la solution et on traite comme décrit en a).

On obtient après évaporation du solvant un solide, F: 152–156 °C; Rendement: 86%.

Chromatographie en couche mince:

1 seule tache de Rf = 0,55 (acétate d'éthyle – méthanol 9–1, vol/vol).

d) – Boc – Tyr – Gly – Trp – Met – Asp (β O Bzl) – $NH_2$

Le produit obtenu au paragraphe c) (0,71 g) est traité par 3,5 ml d'acide trifluoracétique pendant 30 minutes à température ambiante. Par addition d'éther, on précipite une poudre blanche qu'on essore, lavec avec de l'éther et sèche sous vide en présence de potasse.

Ce solide est dissous dans le diméthylformamide (10 ml) et on ajoute 0,4 g de Boc – Tyr – ONp et 0,18 ml de DIEA. Après 8 heures de réaction, on traite comme indiqué au paragraphe c). On chromatographie sur une colonne de gel de silice en éluant avec un mélange acétate d'éthyle-méthanol, 9–1 (vol/vol) pour obtenir un solide, F: 128–135 °C; Rendement: 72%.

Chromatographie en couche mince:

1 seule tache de Rf = 0,4 (acétate d'éthyle-méthanol, 95–5, vol/vol).

e) – Boc – Tyr – Gly – Trp – Met – Asp – $NH_2$

0,2 g du produit obtenu ci-dessus sont dissous dans un mélange diméthylformamide-eau-DIEA (8–1–1, vol/vol) et on hydrogène en présence de palladium sur sulfate de baryum à 10% (0,050 g) pendant 12 heures. On filtre le catalyseur et évapore les solvants à siccité sous vide à température inférieure à 50 °C.

Le résidu est repris dans 20 ml d'acétate d'éthyle et 20 ml de solution d'ammoniaque à 5%. On agite et décante. La phase aqueuse est acidifiée par addition d'acide citrique solide et extraite 2 fois avec de l'acétate d'éthyle. Les extraits organiques réunis sont lavés avec de l'eau, séchés et concentrés à siccité sous vide. On obtient une poudre blanche, F: 210 °C (déc); Rendement: 56%.

Chromatographie en couche mince:

Rf = 0,4 (acétate d'éthyle 9 – méthanol 1 – acide acétique 0,5, vol/vol).

Exemple 5

Z – Glu – Ala – Tyr – Gly – Trp – Met – Asp – $NH_2$

R = Z; A = Glu – Ala – Tyr; B = Met

A) – Z – Glu (γ O But) – Ala – Tyr – Gly – OH

a) – Boc – Gly – Cam

Ce produit est préparé selon la méthode de GISIN par action de l'alpha chloracétamide sur la Boc-glycine au sein du diméthylformamide à 40–50 °C durant 24 heures.

Rendement: 80% environ; F: 64–67 °C (éther).

b) – Boc – Tyr – Gly – Cam

On dissout 2,4 g de Boc-Gly-Cam dans 10 ml d'acide trifluoro-acétique et laisse 30 minutes. On ajoute 150 ml d'éther, essore le précipité, lave avec de l'éther plusieurs fois et sèche.

On dissout ce produit dans 20 ml de diméthylformamide et ajoute 3,22 g de Boc – Tyr – ONp. On refroidit à 0 °C et ajoute 1,3 ml de DIEA. On laisse 12 heures à température ambiante puis on évapore à siccité sous vide en maintenant la température inférieure à 50 °C. On reprend le résidu dans 200 ml d'acétate d'éthyle, lave avec une solution de bicarbonate de sodium, puis avec de

l'eau, avec une solution à 10% d'acide citrique et à nouveau avec de l'eau.

On sèche sur sulfate de sodium et évapore à siccité sous vide. On obtient un solide.

F: 113–116 °C $[\alpha]_D^{20} = -9{,}2$ (C=1, diméthylformamide). Rendement: 85%.

c) – Boc – Ala – Tyr – Gly – Cam

On laisse 1/2 heure à température ambiante la solution de 3,55 g du dipeptide obtenu ci-dessus dans 15 ml d'acide trifluoroacétique. On ajoute 200 ml d'éther et essore le précipité qu'on lave plusieurs fois avec de l'éther et sèche.

On dissout le solide dans 15 ml de diméthylformamide et ajoute 2,63 g de Boc – Ala – ONp et 1,7 ml de DIEA. Après 12 heures à température ambiante, on traite le mélange réactionnel comme indiqué au paragraphe b).

On isole le produit attendu;

F: 129–132 °C $[\alpha]_D^{20} = -21{,}1$ (C=1, diméthylformamide). Rendement: 80%.

d) – Z – Glu (γ O But) – Ala – Tyr – Gly – Cam

On traite 0,932 g du tripeptide préparé ci-dessus par 5 ml d'acide trifluoroacétique pendant 30 minutes à température ambiante et isole le tripeptide déprotégé à l'azote par précipitation à l'éther comme indiqué précédemment. On dissout le produit obtenu dans 10 ml de diméthylformamide et ajoute 1,08 g de Z – Glu (γ O But) – ONp et 0,38 ml de DIEA. On laisse 12 heures et traite comme indiqué précédemment.

F: 135–137 °C (acétate d'éthyle-éther, 1–1 vol/vol)

$[\alpha]_D^{20} = -12$ (C=1, diméthylformamide). Rendement: 85%.

e) – Z – Glu (γ O But) – Ala – Tyr – Gly – OH

A la solution de 1,37 g du peptide préparé ci-dessus dans 25 ml de diméthylformamide, on ajoute sous agitation la solution de 0,318 g de carbonate de sodium dans 25 ml d'eau puis laisse 1 heure 30 sous agitation.

On neutralise à pH voisin de 7 par addition d'une solution d'acide citrique à 20% et concentre à siccité sous vide. On dissout le résidu dans 20 ml d'une solution de carbonate de sodium à 20% et on lave la phase aqueuse avec 2 fois 15 ml d'acétate d'éthyle. On acidifie la phase aqueuse à froid par addition d'acide citrique solide. On essore le précipité, lave avec de l'eau et sèche.

F: 184–185 °C $[\alpha]_D^{20} = -12$ (C=1, diméthylformamide). Rendement: 75%.

B) – FMOC – Trp – Met – Asp (β O But) – $NH_2$

a) – FMOC – Met – Asp (β O But) $NH_2$

On dissout à froid dans 50 ml de diméthylformamide 5 g de HCl, Asp (β O But) – $NH_2$, 7,42 g de FMOC méthionine, 8,84 g de BOP.

On ajoute 7,8 ml de DIEA et agite pendant 12 heures à température ambiante. On évapore à siccité sous vide à température inférieure à 50 °C. On reprend le résidu dans un mélange acétate d'éthyle-éther et laisse cristalliser.

F: 184–185 °C $[\alpha]_D^{20} = -9{,}1$ (C=1, diméthylformamide). Rendement: 87%.

b) – FMOC – Trp – Met – Asp (β O But) – $NH_2$

On dissout 5,41 g du dipeptide protégé obtenu ci-dessus dans 150 ml de diméthylformamide contenant 15 ml de diéthylamine et laisse 2 heures sous agitation à température ambiante.

On évapore à siccité sous vide et reprend le résidu dans 20 ml de diméthylformamide. On ajoute cette solution à une solution contenant 3,82 g de FMOC – Trp, 3,98 g de BOP et 3,4 ml de DIEA dans 20 ml de diméthylformamide. On agite 12 heures à température ambiante et traite comme indiqué au paragraphe précédent en cristallisant dans l'acétate d'éthyle.

F: 175 °C (décomposition) $[\alpha]_D^{20} = -23$ (C=1, diméthylformamide), Rendement: 75%.

C) – Z – Glu – Ala – Tyr – Gly – Trp – Met – Asp – $NH_2$

a) – Z – Glu (α O But) – Ala – Tyr – Gly – Trp – Met – Asp (β O But) – $NH_2$

On dissout 5 g du tripeptide protégé de l'exemple 5 B – b) dans 100 ml de diméthylformamide contenant 10 ml de diéthylamine et laisse 2 heures sous agitation à température ambiante. On évapore à siccité et reprend le résidu dans 20 ml de diméthylformamide. On ajoute 1,26 g du tétrapeptide protégé obtenu à l'exemple 5 A – e), 0,84 g de BOP et 0,69 ml de DIEA, et agite pendant 12 heures à température ambiante. On évapore à siccité sous vide et triture le résidu dans l'acétate d'éthyle. On filtre et lave avec une solution saturée de carbonate de sodium, avec de l'eau, avec une solution d'acide citrique à 20%, avec de l'eau et finalement avec de l'acétate d'éthyle. On sèche sous vide.

F: 210 °C (décomposition) $[\alpha]_D^{20} = -23$ (C=1, diméthylformamide). Rendement: 72%.

b) – Déprotection

On dissout 1,1 g de l'heptapeptide protégé obtenu ci-dessus dans 10 ml d'acide trifluoroacétique contenant 1 ml de thioanisole. On laisse 1 heure à température ambiante puis on ajoute 200 ml d'éther. On essore le solide blanc qui s'est séparé et on lave plusieurs fois avec de l'éther puis sèche sous vide.

F: 210 °C (décomposition) $[\alpha]_D^{20} = -24$ (C=1, diméthylformamide). Rendement: 97%.

Essais pharmacologiques

Les composés selon l'invention ont été étudiés en ce qui concerne leurs propriétés thérapeutiques. Plus particulièrement, ces composés ont été

étudiés in vivo en ce qui concerne leur effet sécrétoire gastrique chez le rat.

Le modèle choisi pour la mesure de l'effet sécrétoire est celui de l'estomac de rat anesthésié reperfusé. Le protocole suivi est une modification de celui d'écrit préalablement par Ghos et Schild.

Un rat mâle de souche Wistar de 300 g, à jeun de 18 heures, est anesthésié à l'uréthane (solution à 10%, 1,5 ml/100 g i.p.). On pratique ensuite une trachéotomie et un cathétérisme de la veine du pénis qui permettra l'administration i.v. des peptides. On place ensuite une canule dans l'oesophage jusqu'au cardia et une seconde dans le duodénum (par une duodénotomie effectuée à 3 cm environ du pylore) jusque dans la zone antrale gastrique.

Un soluté propionique-succinique (pH 5,5) qui donne une variaton linéaire du pH en fonction de la concentration en ions $H^+$ est utilisé pour perfuser l'estomac en circuit ouvert ou fermé avec un débit de 3 ml/min. La température corporelle, ainsi que celle du soluté, est contrôlée et maintenue à 30 °C. La sécrétion acide de l'estomac va entraîner une variation de pH détectée par l'électrode de verre et enregistrée en fonction du temps. Après stabilisation de la sécrétion basale, on injecte la gastrine par voie intraveineuse soit en perfusion, soit en injection unique. La réponse est enregistrée en fonction du temps et la quantité d'acide sécrété est mesurée sur l'enregistrement par différence avec la sécrétion basale.

La même expérience est réalisée soit en injectant le peptide à étudier i.v. sur un plateau de sécrétion acide stimulée par la gastrine, soit en associant le peptide avec le stimulant dans des rapports de concentration variables. Enfin, le peptide est administré seul à différentes doses pour examiner son effet agoniste.

Les expériences effectuées avec les produits de l'exemple 3: Z – Tyr (O $SO_3H$) – Met – Gly – Trp – Met – Asp – $NH_2$ et de l'exemple 4: Boc – Tyr – Gly – Trp – Met – Asp – $NH_2$ ont donné les résultats suivants.

A) Effet agoniste

| Peptide | Dose | Effet sécrétoire (40 minutes) |
|---|---|---|
| Exemple 3 | 1 000 µg/kg | 0,15 µ mol $H^+$ |
| Exemple 4 | 5 000 µg/kg | 0,1 µ mol $H^+$ |
| Exmple 5 | 10 000 µg/kg | 0 |
| Gastrine (HG 13) | 0,5 µg/kg | 16 µ mol $H^+$ |

B) Effet antagoniste

| Peptide | Dose d'agoniste (minigastrine) | Dose d'antagoniste | Effet inhibiteur % |
|---|---|---|---|
| Exemple 3 | 1,5 µg/kg/h (perfusion) | 1,5 mg/kg | 47,4 |
| | 0,6 µg/kg (unique) | 1,5 mg/kg | 47 |
| Exemple 4 | 0,6 µg/kg (unique) | 1,2 mg/kg | 50,4 |
| Exemple 5 | 0,6 µg/kg (unique) | 1,5 mg/kg | 50 |

D'après les résultats, on constate que:

– les composés selon l'invention présentent un effet agoniste extrêmement faible vis-à-vis de la gastrine et ce malgré la valeur élevée des doses utilisés;

– les composés selon l'invention présentent un effet inhibiteur important sur la sécrétion gastrique, de l'ordre de 50% dans les conditions expérimentales utilisées.

Par suite, les composés selon l'invention pourront être utilisés en thérapeutique humaine dans tous les cas où il est utile de diminuer la sécrétion gastrique et en particulier pour le traitement des ulcères gastro-duodénaux.

Les composés de la présente invention peuvent être utilisés de préférence par voie injectable: intraveineuse, intramusculaire ou sous-cutanée. Ils sont utilisés dans un solvant tel que le sérum physiologique (solution saline isotonique).

La posologie peut varier suivant l'intensité de l'effet thérapeutique recherchée, la gravité de l'affection à traiter et la voie d'administration utilisée. Elle doit donc être déterminée pour chaque patient en fonction de ces divers critères. Le plus souvent elle est comprise entre 1 et 100 mg de principe actif par kg de poids corporel.

**Revendications pour les Etats contractants BE, CH, DE, FR, GB, IT, LI, LU, NL, SE**

1. Peptides de formule générale

$$R - A - Gly - Trp - B - Asp\,NH_2 \qquad (I)$$

dans laquelle:

– Asp $NH_2$ représente l'amide en α de l'acide aspartique de formule:

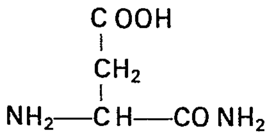

– R représente l'hydrogène, un groupe protecteur de la fonction amine terminale tel que t.butyloxy carbonyl (BOC), benzyloxy carbonyl (Z), alcanoyle inférieur

– A désigne

– soit la tyrosine, la tyrosine-O-sulfate, la thréonine ou la méthionine;

– soit un dipeptide choisi parmi: – Ala – TYR –, – TYR – Thr –, – TYR – Met –, en désignant par TYR l'ensemble des 2 amino-acides constitué par la tyrosine ou la tyrosine-O-sulfate;

– soit un tripeptide choisi parmi: – Glu – Ala – TYR –, – Asp – TYR – Thr –, – Asp – TYR – Met –;

– soit un tétrapeptide choisi parmi: – Glu – Glu – Ala – TYR –, – Gln – Asp – TYR – Thr –, – Arg – Asp – TYR – Met –;

– soit un pentapeptide choisi parmi: – Glu – Glu – Glu – Ala – TYR –, – Pyr – Gln – Asp – TYR – Thr –, – Asp – Arg – Asp – TYR – Met-.

– B désigne la méthionine, la leucine ou la norleucine.

2. Peptide selon la revendication 1 de formule H – Met – Gly – Trp – Met – Asp $NH_2$

3. Peptide selon la revendication 1 de formule R – Tyr – Met – Gly – Trp – Met – Asp – $NH_2$,

4. Peptide selon la revendication 1 de formule R – $Tyr(SO_3H)$ – Met – Gly – Trp – Met – Asp-$NH_2$.

5. Peptide selon la revendication 1 de formule R – Tyr – Gly – Trp – Met – Asp – $NH_2$.

6. Peptide selon la revendication 1 de formule: – Glu – Ala – Tyr – Gly – Trp – Met – Asp – $NH_2$.

7. Procédé de préparation des peptides selon l'une des revendications 1 à 6, caractérisé en ce que:

– on utilise comme produit de départ l'acide aspartique α-amide

– on réalise successivement sur ledit acide les couplages des divers acides aminés en utilisant un ester activé desdits acides, la réaction étant réalisée au sein du diméthylformamide et en présence d'un composé choisi parmi la diisopropyléthylamine et l'hydroxy-1 benzotriazole, lesdits acides aminés ayant été au préalable convernablement protégés sur leur fonction amine en α.

8. Médicaments utiles notamment comme inhibiteurs de la sécrétion gastrique, caractérisés en ce qu'ils contiennent au moins un peptide selon les revendications 1 à 6.

**Revendications pour l'Etat contractant: AT**

1. Procédé pour l'obtention de peptides de formule générale

R – A – Gly – Trp – B – Asp $NH_2$ I

dans laquelle:

– Asp $NH_2$ représente l'amide en α de l'acide aspartique de formule:

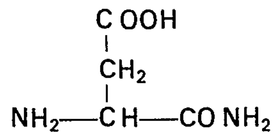

– R représente l'hydrogène, un groupe protecteur de la fonction amine terminale tel que t.butyloxy carbonyl (BOC), benzyloxy carbonyl (Z), alcanoyle inférieure;

– A désigne

– soit la tyrosine, la tyrosine-O-sulfate, la thréonine ou la méthionine;

– soit un dipeptide choisi parmi: – Ala – TYR –, – TYR – Thr –, – TYR – Met –, en désignant par TYR l'ensemble des 2 amino-acides constitué par la tyrosine ou la tyrosine-O-sulfate;

– soit un tripeptide choisi parmi: – Glu – Ala – TYR –, – Asp – TYR – Thr –, – Asp – TYR – Met –;

– soit un tétrapeptide choisi parmi: – Glu – Glu – Ala – TYR –, – Gln – Asp – TYR – Thr –, – Arg – Asp – TYR – Met –;

– soit un pentapeptide choisi parmi: – Glu – Glu – Glu – Ala – TYR –, – Pyr – Gln – Asp – TYR – Thr –, – Asp – Arg – Asp – TYR – Met –.

– B désigne la méthionine, la leucine ou la norleucine, caractérisé en ce que:

– on utilise comme produit de départ l'acide aspartique α-amide;

– on réalise successivement sur ledit acide les couplages des divers acides aminés en utilisant un ester activé desdits acides, la réaction étant réalisée au sein du diméthylformamide et en présence d'un composé choisi parmi la diisopropyléthylamine et l'hydroxy-1 benzotriazole, lesdits acides aminés ayant été au préalable convenablement protégés sur leur fonction amine en α.

2. Utilisation des peptides de formule:

R – A – Gly – Trp – B – Asp $NH_2$ I

dans laquelle:

– Asp $NH_2$ représente l'amide en α de l'acide aspartique de formule:

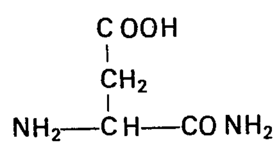

– R représente l'hydrogène, un groupe protecteur de la fonction amine terminale tel que t.butyloxy carbonyl (BOC), benzyloxy carbonyl (Z), alcanoyle inférieur;

– A désigne

– soit la tyrosine, la tyrosine-O-sulfate, la thréonine ou la méthionine;

– soit un dipeptide choisi parmi: – Ala – TYR –, – TYR – Thr –, – TYR – Met –, en désignant par

TYR l'ensemble des 2 amino-acides constitué par la tyrosine ou la tyrosine-O-sulfate;

– soit un tripeptide choisi parmi: – Glu – Ala – TYR –, – Asp – TYR – Thr –, – Asp – TYR – Met –;

– soit un tétrapeptide choisi parmi: – Glu – Glu – Ala – TYR –, – Gln – Asp – TYR – Thr –, – Arg – Asp – TYR – Met –;

– soit un pentapeptide choisi parmi: – Glu – Glu – Glu – Ala – TYR –, – Pyr – Gln – Asp – TYR – Thr –, – Asp – Arg – Asp – TYR – Met –.

– B désigne la méthionine, la leucine ou la norleucine; pour la préparation de médicaments utiles notamment comme inhibiteurs de la sécrétion gastrique.

**Patentansprüche für die Vertragsstaaten BE, CH, DE, FR, GB, IT, LI, LU, NL, SE:**

1. Peptide der allgemeinen Formel

R – A – Gly – Trp – B – Asp $NH_2$ (I),

worin:

– Asp $NH_2$ das α-Amid der Asparaginsäure der Formel:

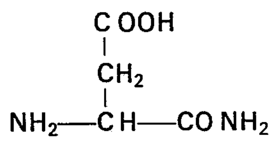

darstellt,

– R für Wasserstoff, eine Schutzgruppe der terminalen Aminfunktion, wie tert.Butyloxycarbonyl (BOC), Benzyloxycarbonyl (Z), Niedrigalkanoyl, steht,

– A folgendes bedeutet:

– entweder Tyrosin, Tyrosin-O-sulfat, Threonin oder Methionin;

– oder ein Dipeptid, ausgewählt aus: – Ala – TYR –, – TYR – Thr –, – TYR – Met –, wobei mit TYR die Gruppe der 2-Aminosäuren, gebildet durch Tyrosin oder Tyrosin-O-sulfat, bezeichnet ist;

– oder ein Tripeptid, ausgewählt aus: – Glu – Ala – TYR –,– Asp – TYR – Thr –, Asp – TYR – Met –;

– oder ein Tetrapeptid, ausgewählt aus: – Glu – Glu – Ala – TYR –, Gln – Asp – TYR – Thr –, – Arg – Asp – TYR – Met –;

– oder ein Pentapeptid, ausgewählt aus: – Glu – Glu – Glu – Ala – TYR –, – Pyr – Gln – Asp – Tyr – Thr –, – Asp – Arg – Asp – TYR – Met –;

– B Methionin, Leucin oder Norleucin darstellt.

2. Peptid nach Anspruch 1 der Formel H – Met – Gly – Trp – Met – Asp–$NH_2$.

3. Peptid nach Anspruch 1 der Formel R – Tyr – Met – Gly – Trp – Met – Asp–$NH_2$.

4. Peptid nach Anspruch 1 der Formel R – Tyr-($SO_3H$) – Met – Gly – Trp – Met – Asp – $NH_2$.

5. Peptid nach Anspruch 1 der Formel R – Tyr – Gly – Trp – Met – Asp-$NH_2$.

6. Peptid nach Anspruch 1 der Formel Z – Glu – Ala – Tyr – Gly – Trp – Met – Asp – $NH_2$.

7. Verfahren zur Herstellung der Peptide nach einem der Ansprüche 1 bis 6, dadurch gekennzeichnet, dass:

– man als Ausgangsprodukt α-Amid-asparaginsäure einsetzt,

– man nacheinander an dieser Säure die Kopplungen der verschiedenen Aminosäuren unter Verwendung eines aktivierten Esters dieser Säuren vornimmt, wobei die Reaktion in Dimethylformamid und in Gegenwart einer Verbindung, ausgewählt aus Diisopropyläthylamin und 1-Hydroxy-benzotriazol, durchgeführt wird, wobei die Aminosäuren zuvor an ihrer α-Aminfunktion entsprechend geschützt wurden.

8. Medikamente, geeignet insbesondere als Inhibitoren der Magensekretion, dadurch gekennzeichnet, dass sie mindestens ein Peptid nach den Ansprüchen 1 bis 6 enthalten.

**Patentansprüche für den Vertragsstaat AT:**

1. Verfahren zur Herstellung von Peptiden der allgemeinen Formel

R – A – Gly – Trp – B – Asp $NH_2$ (I),

worin:

– Asp $NH_2$ das α-Amid der Asparaginsäure der Formel:

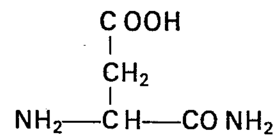

darstellt,

– R für Wasserstoff, eine Schutzgruppe der terminalen Aminfunktion, wie tert.Butyloxycarbonyl (BOC), Benzyloxycarbonyl (Z), Niedrigalkanoyl, steht,

– A folgendes bedeutet:

– entweder Tyrosin, Tyrosin-O-sulfat, Threonin oder Methionin;

– oder ein Dipeptid, ausgewählt aus: – Ala – TYR –, – TYR – Thr –, – TYR – Met –, wobei mit TYR die Gruppe der 2-Aminosäure, gebildet durch Tyrosin oder Tyrosin-O-sulfat, bezeichnet ist;

– oder ein Tripeptid, ausgewählt aus: – Glu – Ala – TYR –, – Asp – TYR – Thr –, Asp – TYR – Met –;

– oder ein Tetrapeptid, ausgewählt aus: – Glu – Glu – Ala – TYR –, Gln – Asp – Tyr – Thr –, – Arg – Asp – TYR – Met –;

– oder ein Pentapeptid, ausgewählt aus: – Glu – Glu – Glu – Ala – TYR –, – Pyr – Gln – Asp – TYR – Thr –, – Asp – Arg – Asp – TYR – Met –;

– B Methionin, Leucin oder Norleucin darstellt, dadurch gekennzeichnet, dass:

– man als Ausgangsprodukt α-Amid-asparaginsäure einsetzt,

– man nacheinander an dieser Säure die Kopplungen der verschiedenen Aminosäure unter Verwendung eines aktivierten Esters dieser Säuren vornimmt, wobei die Reaktion in Dimethylformamid und in Gegenwart einer Verbindung, ausgewählt aus Diisopropyläthylamin und 1-Hydroxybenzotriazol, durchgeführt wird, wobei die Aminosäuren zuvor an ihrer α-Aminfunktion entsprechend geschützt wurden.

2. Verwendung der Peptide der Formel

R – A – Gly – Trp – B – Asp $NH_2$ (I),

worin:

– Asp $NH_2$ das α-Amid der Asparaginsäure der Formel:

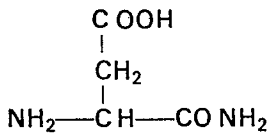

darstellt,

– R für Wasserstoff, eine Schutzgruppe der terminalen Aminfunktion, wie tert. Butyloxycarbonyl (BOC), Benzyloxycarbonyl (Z), Niedrigalkanoyl, steht,

– A folgendes bedeutet:

– entweder Tyrosin, Tyrosin-O-sulfat, Threonin oder Methionin;

– oder ein Dipeptid, ausgewählt aus: – Ala – TYR –, – TYR – Thr –, – TYR – Met –, wobei mit TYR die Gruppe der 2-Aminosäure, gebildet durch Tyrosin oder Tyrosin-O-sulfat, bezeichnet ist;

– oder ein Tripeptid, ausgewählt aus: – Glu – Ala – TYR –, – Asp – TYR – Thr –, Asp – TYR – Met –;

– oder ein Tetrapeptid, ausgewählt aus: – Glu – Glu – Ala – TYR –, Gln – Asp – TYR – Thr –, – Arg – Asp – TYR – Met –;

– oder ein Pentapeptid, ausgewählt aus: – Glu – Glu – Glu – Ala – TYR –, – Pyr – Gln – Asp – TYR – Thr –; – Asp – Arg – Asp – TYR – Met –;

– B Methionin, Leucin oder Norleucin darstellt, zur Herstellung von Medikamenten, die insbesondere als Inhibitoren der Magensekretion geeignet sind.

**Claims for the contracting states: BE, CH, DE, FR, GB, IT, LI, LU, NL, SE**

1. Peptides of general formula:

R – A – Gly – Trp – B – Asp – $NH_2$ (I)

in which:

– Asp $NH_2$ represents the amide in α position of the aspartic acid of formula:

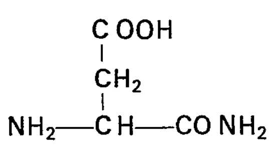

– R represents hydrogen, a protector group of the terminal amine function such as t.butyloxy carbonyl (BOC), benzyloxy carbonyl (Z), lower alkanoyl,

– A designates:

– either tyrosine, tyrosine-O-sulfate, threonine or methionine;

– or a dipeptide selected from: – Ala – TYR –, – TYR – Thr –, – TYR – Met –, TYR designating the ensemble of the 2 amino-acids constituted by tyrosine or tyrosine-O-sulfate;

– or a tripeptide selected from: – Glu – Ala – TYR –, Asp – TYR – Thr –, – Asp – TYR – Met –;

– or a tetrapeptide selected from: – Glu – Glu – Ala – Tyr –, – Gln – Asp – TYR – Thr –, – Arg – Asp – TYR – Met –;

– or a pentapeptide selected from: – Glu – Glu – Glu – Ala – TYR –; – Pyr – Gln – Asp – TYR – Thr –, – Asp – Arg – Asp – TYR – Met –;

– B designates methionine, leucine or norleucine.

2. A peptide according to claim 1 of formula H – Met – Gly – Trp – Met – Asp – $NH_2$.

3. A peptide according to claim 1 of formula: R – Tyr – Met – Gly – Trp – Met – Asp – $NH_2$.

4. A peptide according to claim 1 of formula: R – Tyr($SO_3H$) – Met – Gly – Trp – Met – Asp–$NH_2$.

5. A peptide according to claim 1 of formula: R –Tyr – Gly – Trp – Met – Asp – $NH_2$.

6. A peptide according to claim 1 of formula: Z – Glu – Ala – Tyr – Gly – Trp – Met – Asp – $NH_2$.

7. A process for preparing the peptides according to any one of claims 1 to 6, characterized in that it comprises the steps of:

– using α-amide aspartic acid as starting product,

– successively effecting on said acid the couplings of the various amino acids by using an activated ester of said acids, the reaction being carried out within dimethylformamide and in the presence of a compound selected from diisopropylethylamine and 1-hydroxy benzotriazole, said amino acids having been previously appropriately protected on their amine function in α position.

8. Drugs useful in particular as inhibitors of gastric secretion, characterized in that they contain at least one peptide according to claims 1 to 6.

**Claims for the contracting state: AT**

1. Process for preparing the peptides of general formula:

R – A – Gly – Trp – B – Asp – $NH_2$ (I)

in which:

– Asp $NH_2$ represents the amide in α position of the aspartic acid of formula:

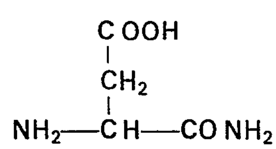

– R represents hydrogen, a protector group of

the terminal amine function such as t-butyloxy carbonyl (BOC), benzyloxy carbonyl (Z), lower alkanoyl,

– A designates:

– either tyrosine, tyrosine-O-sulfate, threonine or methionine;

– or a dipeptide selected from: – Ala – TYR –, – TYR – Thr –, – TYR – Met –, TYR designating the ensemble of the 2 amino-acids constituted by tyrosine or tyrosine-O-sulfate;

– or a tripeptide selected from: – Glu – Ala – TYR –, Asp – TYR – Thr –, – Asp – TYR – Met –;

– or a tetrapeptide selected from: – Glu – Glu – Ala – TYR –, – Gln – Asp – TYR – Thr –, – Arg – Asp – TYR – Met –;

– or a pentapeptide selected from: – Glu – Glu – Glu – Ala – TYR –, – Pyr – Gln – Asp – TYR – Thr –, – Asp – Arg – Asp – TYR – Met –;

– B designates methionine, leucine or norleucine, characterized in that it comprises the steps of:

– using α-amide aspartic acid as starting product,

– successively effecting on said acid the couplings of the various amino acids by using an activated ester of said acids, the reaction being carried out within dimethylformamide and in the presence of a compound selected from diisopropylethylamine and 1-hydroxy benzotriazole, said amino acids having been previously appropriately protected on their amine function in α position.

2. Use of the peptides of general formula:

$$R - A - Gly - Trp - B - Asp - NH_2 \qquad (I)$$

in which:

– Asp $NH_2$ represents the amide in α position of the aspartic acid of formula:

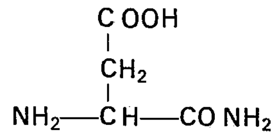

– R represents hydrogen, a protector group of the terminal amine function such as t.butyloxy carbonyl (BOC), benzyloxy carbonyl (Z), lower alkanoyl,

– A designates:

– either tyrosine, tyrosine-O-sulfate, threonine or methionine;

– or a dipeptide selected from: – Ala – TYR –, – TYR – Thr –, – TYR – Met –, TYR designating the ensemble of the 2 amino-acids constituted by tyrosine or tyrosine-O-sulfate;

– or a tripeptide selected from: – Glu – Ala – TYR –, Asp – TYR – Thr –, – Asp – TYR – Met –;

– or a tetrapeptide selected from: – Glu – Glu – Ala – TYR –, – Gln – Asp – TYR – Thr –, – Arg – Asp – TYR – Met –;

– or a pentapeptide selected from: – Glu – Glu – Glu – Ala – TYR –, – Pyr – Gln – Asp – TYR – Thr –, – Asp – Arg – Asp – TYR – Met –;

– B designates methionine, leucine or norleucine, for the preparation of drugs useful in particular as inhibitors of gastric secretion.